# EUROPEAN PATENT APPLICATION

(11) **EP 3 982 698 A1**
(43) Date of publication of application: **13.04.2022**
(21) Application number: 20818108.1
(22) Date of filing: 01.06.2020
(51) Int. Cl.: H05H 1/26, A61B 18/04

(54) **PLASMA IRRADIATION APPARATUS AND TIP DEVICE**

(30) Priority: 04.06.2019 JP 2019104428
(71) Applicant: NGK SPARK PLUG CO., LTD., Mizuho-ku Nagoya-shi, Aichi 467-8525 (JP)
(72) Inventor: ITOH Shinsuke, Nagoya-shi, Aichi 467-8525 (JP)
(74) Representative: Zimmermann & Partner Patentanwälte mbB
(86) International application number: PCT/JP2020/021654
(87) International publication number: WO 2020/246435

(57) **Abstract**

Disclosed is a plasma irradiation apparatus (20, 320, 420, 520, 620, 720, 820) with a gas guide channel (30) and a creeping discharge section (40, 340, 440, 540, 640, 740, 840). The creeping discharge section (40, 340, 440, 540, 640, 740, 840) is disposed such that one of a discharge electrode and a ground electrode faces a flow path (36) directly or via another member, and is configured to generate creeping discharge in the flow path (36) by the application of a periodically changing voltage to the discharge electrode. At least a part of the ground electrode is arranged closer to an outlet port (34) than the discharge electrode.

## Description

### Technical Field

The present invention relates to a plasma irradiation apparatus and a distal device.

### Background Art

Patent Document 1 discloses a plasma surface treatment apparatus that generates creeping-discharge-type plasma discharge. This apparatus has a reaction part structural body formed with a flow path for a plasma generating gas and creeping discharge generation electrodes disposed on a wall surface of the flow path. The creeping discharge generation electrodes include a discharge electrode located on a lateral side of the flow path in a direction of travel of the plasma generating gas and an induction electrode opposed to the discharge electrode via a dielectric member.

### Prior Art Documents

### Patent Document

Patent Document 1: Japanese Laid-Open Patent Publication No. 2005-50723

### Summary of the Invention

### Problems to be Solved by the Invention

By the way, it is known that plasma is likely to be generated in the vicinity of the creeping discharge generation electrodes. By bringing the creeping discharge generation electrodes closer to an outlet port of the flow path, it becomes possible to emit a greater amount of plasma from the outlet port of the flow path to the plasma irradiation target. From another point of view, it becomes possible by bringing the creeping discharge generation electrodes closer to the outlet port of the flow path to efficiently emit plasma to the plasma irradiation target and decrease the voltage applied between the creeping discharge generation electrodes. In the apparatus of Patent Document 1, however, a front end of the discharge electrode and a front end of the induction electrode are arranged at substantially the same position in the direction of travel of the plasma generating gas (i.e. in the direction of extension of the flow path). In such an arrangement, the distance between the discharge electrode, which largely changes in potential, and the plasma irradiation target becomes short when the creeping discharge generation electrodes are brought closer to the outlet port of the flow path so as to obtain the above effects. There thus arises a possibility that the plasma irradiation target may be electrically adversely affected.

It is conceivable to bring the creeping discharge generation electrodes further away from the outlet port of the flow path in order to prevent the plasma irradiation target from being electrically adversely affected. In this case, however, there arises a possibility that it may not be possible to sufficiently emit plasma from the outlet port of the flow path to the plasma irradiation target or may not be possible to decrease the voltage applied between the creeping discharge generation electrodes.

The present invention has been accomplished to solve at least part of the above-mentioned problems. It is an object of the present invention to provide a technique for enabling a creeping-discharge-type plasma irradiation apparatus to irradiate a plasma irradiation target with a greater amount of plasma, or irradiate a plasma irradiation target with plasma efficiently and decrease a voltage applied to a discharge electrode, and to prevent the plasma irradiation target from being electrically adversely affected by the discharge electrode whose potential changes largely.

### Means for Solving the Problems

According to one aspect of the present invention, there is provided a plasma irradiation apparatus, comprising:
a gas guide channel having an inlet port for introducing a gas, an outlet port for discharging the gas and a flow path located between the inlet port and the outlet port such that the gas introduced from the inlet port flows to the outlet port through an inner space of the flow path; and
a creeping discharge section having a dielectric layer, a discharge electrode and a ground electrode opposed to the discharge electrode with the dielectric layer interposed between the discharge electrode and the ground electrode, the creeping discharge section being disposed such that one of the discharge electrode and the ground electrode faces the flow path directly or via another member and being configured to generate creeping discharge in the flow path by the application of a periodically changing voltage to the discharge electrode,
wherein at least a part of the ground electrode is arranged closer to the outlet port than the discharge electrode.

In the above-configured plasma irradiation apparatus, at least a part of the ground electrode is arranged closer to the outlet port than the discharge electrode. In this arrangement, the ground electrode is brought closer to the outlet port; whereas the discharge electrode is brought further away from the outlet port. Thus, plasma can be generated in the vicinity of the ground electrode brought closer to the outlet port. In other words, plasma is easily generated in an area close to the outlet port. Especially when such an effect is achieved in the case of creeping discharge type, it is possible to irradiate the plasma irradiation target with a greater amount of plasma or irradiate the plasma irradiation target with plasma more efficiently by the application of a lower voltage. Since the discharge electrode is located away from the outlet port, it is possible to prevent the plasma irradiation target from being electrically adversely affected by the discharge electrode.

In the above-configured plasma irradiation apparatus, the gas guide channel may have the outlet port on one side thereof in a first direction and the inlet port on the other side thereof in the first direction. Further, the flow path may include a first flow passage extending along the first direction and a second flow passage disposed at a location downstream of the first flow passage and closer to the outlet port than the first flow passage in the first direction and having a width narrower than that of the first flow passage.

In this plasma irradiation apparatus, the gas is allowed to efficiently flow to the outlet port along the first direction. As the second flow passage of relatively narrow width is arranged downstream of the first flow passage, the flow rate of the gas in the vicinity of the outlet port is increased. It thus becomes easy to, when the outlet port is directed to the plasma irradiation target, efficiently emit the plasma to the plasma irradiation target.

In the above-configured plasma irradiation apparatus, at least a part of the ground electrode may be situated in the arrangement area of the second flow passage in the first direction.

In this plasma irradiation apparatus, the ground electrode is situated in the arrangement area of the relatively narrow width second flow passage so that plasma is more easily generated in the second flow passage in which the flow rate of the gas is increased. It thus becomes easy to more efficiently emit the plasma generated in the flow path.

In the above-configured plasma irradiation apparatus, the discharge electrode may be situated only in the arrangement area of the first flow passage in the first direction.

In this plasma irradiation apparatus, the discharge electrode is arranged in the area relatively far away from the outlet port in the first direction (i.e. in the arrangement area of the first flow passage) so that it is possible to, when the outlet port is directed to the plasma irradiation target, reliably prevent the plasma irradiation target from being electrically adversely affected by the discharge electrode.

In the above-configured plasma irradiation apparatus, the discharge electrode may face the flow path directly or via another member and, when viewed in a plane direction perpendicular to the first direction, be displaced in position from the opening area of the outlet port.

In this plasma irradiation apparatus, the discharge electrode whose potential changes largely is arranged to face the flow path. In such an arrangement, there is a possibility that the plasma irradiation target may be electrically adversely affected by the discharge electrode through the inner space of the flow path. However, the arrangement direction of the discharge electrode is displaced from the opening area of the outlet port in the plane direction. In other words, the discharge electrode is hidden when the flow path is seen from the outlet port. It is thus possible to reliably prevent the plasma irradiation target from being electrically adversely affected by the discharge electrode through the inner space of the flow path.

In the above-configured plasma irradiation apparatus, the discharge electrode may have a width narrower than that of the ground electrode.

The creeping-discharge-type plasma irradiation apparatus is advantageous in that, at the time of applying a periodically changing voltage to the discharge electrode, it is easy to limit the absolute value of the applied voltage. When the absolute value of the applied voltage is too limited, on the other hand, there is a risk that plasma may not be generated favorably. However, the discharge electrode is made relatively narrow by setting the width of the discharge electrode narrower than the width of the ground electrode as mentioned above. With such a configuration, plasma is more easily generated between the discharge electrode and the ground electrode so that it becomes easy to attain both of limitation of the applied voltage and favorable generation of the plasma.

There may be provided a distal device comprising: an acting member having an acting portion that acts on biological tissue; and the above-configured plasma irradiation apparatus, wherein the gas guide channel is disposed to emit the gas from the outlet port toward the acting portion.

The above-mentioned distal device enables plasma generation at a location closer to the biological tissue on which the acting portion acts. Especially when such an effect is achieved in the case of creeping discharge type, it is possible to irradiate the biological tissue on which the acting portion acts with a greater amount of plasma or irradiate the biological tissue on which the acting portion acts with plasma more efficiently by the application of a lower voltage. It is further possible to, when the acting portion acts on the biological tissue, prevent the biological tissue from being electrically adversely affected.

There may also be provided a distal device comprising: an acting member having an acting portion that acts on biological tissue; a drive section that drives the acting member; and the above-configured plasma irradiation apparatus, wherein the gas guide channel is disposed to emit the gas from the outlet port toward the acting portion; wherein the drive section is an ultrasonic vibration section that generates ultrasonic vibration; and wherein the acting member is configured to, by transmission of the ultrasonic vibration from the ultrasonic vibration section to the acting member, allow the acting portion to vibrate and thereby perform incision action, ablation action or hemostasis action on the biological tissue.

The above-mentioned distal device not only enables incision, ablation action or hemostasis of the biological tissue by means of the acting portion, but also enables plasma generation at a location closer to the biological tissue. Especially when such an effect is achieved in the case of creeping discharge type, it is possible to irradiate the biological tissue on which the acting portion acts with a greater amount of plasma or irradiate the biological tissue on which the acting portion acts with plasma more efficiently by the application of a lower voltage. It is further possible to, when the acting portion acts on the biological tissue, prevent the biological tissue from being electrically adversely affected.

There may be provided a distal device comprising: an acting member having an acting portion that acts on biological tissue; a drive section that drives the acting member; and the above-configured plasma irradiation apparatus, wherein the gas guide channel is disposed to emit the gas from the outlet port toward the acting portion; wherein the drive section is a high-frequency current supply section that supplies a high-frequency current; and wherein the acting member is configured to allow the acting portion to perform incision action, ablation action or hemostasis action on the biological tissue by flow of the high-frequency current from the high-frequency current supply section through the acting member.

The above-mentioned distal device not only enables incision, ablation or hemostasis of the biological tissue by means of the acting portion, but also enables plasma generation at a location closer to the biological tissue. Especially when such an effect is achieved in the case of creeping discharge type, it is possible to irradiate the biological tissue on which the acting portion acts with a greater amount of plasma or irradiate the biological tissue on which the acting portion acts with plasma more efficiently by the application of a lower voltage. It is further possible to, when the acting portion acts on the biological tissue, prevent the biological tissue from being electrically adversely affected.

In the above-mentioned distal device, the acting member, or an assembly member integrally assembled with the acting member, may serve as the ground electrode.

In this case, the ground electrode disposed in a different region from the acting member or assembly member can be reduced or omitted. It is thus possible to simplify the configuration of the distal device in the region different from the acting member or assembly member.

According to another aspect of the present invention, there is provided a distal device, comprising:
an acting member having an acting portion that acts on biological tissue;
a gas guide channel having an inlet port for introducing a gas, an outlet port for discharging the gas and a flow path located between the inlet port and the outlet port such that the gas is introduced from the inlet port and flows to the outlet port through an inner space of the flow path; and
a creeping discharge section having a dielectric layer, a discharge electrode and a ground electrode opposed to the discharge electrode with the dielectric layer interposed between the discharge electrode and the ground electrode, the creeping discharge section being disposed such that one of the discharge electrode and the ground electrode faces the flow path directly or via another member and being configured to generate creeping discharge in the flow path by the application of a periodically changing voltage to the discharge electrode,
the gas guide channel being disposed to emit the gas from the outlet port toward the acting member,
wherein at least a part of the ground electrode is arranged closer to the outlet port than the discharge electrode, and
wherein the acting member, or an assembly member integrally assembled with the acting member, serves as the ground electrode.

The above-mentioned distal device enables plasma generation at a location closer to the biological tissue on which the acting portion acts. Especially when such an effect is achieved in the case of creeping discharge type, it is possible to irradiate the biological tissue on which the acting portion acts with a greater amount of plasma or irradiate the biological tissue on which the acting portion acts with plasma more efficiently by the application of a lower voltage. It is further possible to, when the acting portion acts on the biological tissue, prevent the biological tissue from being electrically adversely affected. Moreover, the ground electrode disposed in a different region from the acting member or assembly member can be reduced or omitted so that it is possible to simplify the configuration of the distal device in the region different from the acting member or assembly member.

According to still another aspect of the present invention, there is provided a distal device, comprising:
an acting member having an acting member that acts on biological tissue;
a drive section that drives the acting member;
a gas guide channel having an inlet port for introducing a gas, an outlet port for discharging the gas and a flow path located between the inlet port and the outlet port such that the gas is introduced from the inlet port and flows to the outlet port through an inner space of the flow path; and
a creeping discharge section having a dielectric layer, a discharge electrode and a ground electrode opposed to the discharge electrode with the dielectric layer interposed between the discharge electrode and the ground electrode, the creeping discharge section being disposed such that one of the discharge electrode and the ground electrode faces the flow path directly or via another member and being configured to generate creeping discharge in the flow path by the application of a periodically changing voltage to the discharge electrode,
the gas guide channel being disposed to emit the gas from the outlet port toward the acting member,
the drive section being an ultrasonic vibration section that generates ultrasonic vibration,
the acting member being configured to, by transmission of the ultrasonic vibration from the ultrasonic vibration section to the acting member, allow the acting portion to vibrate and thereby perform incision action, ablation action or hemostasis action on the biological tissue,
wherein at least a part of the ground electrode is arranged closer to the outlet port than the discharge electrode, and
wherein the acting member, or an assembly member integrally assembled with the acting member, serves as the ground electrode.

The above-mentioned distal device not only enables incision, ablation or hemostasis of the biological tissue by means of the acting portion, but also enables plasma generation at a location closer to the biological tissue. Especially when such an effect is achieved in the case of creeping discharge type, it is possible to irradiate the biological tissue on which the acting portion acts with a greater amount of plasma or irradiate the biological tissue on which the acting portion acts with plasma more efficiently by the application of a lower voltage. It is further possible to, when the acting portion acts on the biological tissue, prevent the biological tissue from being electrically adversely affected. Moreover, the ground electrode disposed in a different region from the acting member or assembly member can be reduced or omitted so that it is possible to simplify the configuration of the distal device in the region different from the acting member or assembly member.

According to yet another aspect of the present invention, there is provided a distal device, comprising:
an acting member having an acting portion that acts on biological tissue;
a drive section that drives the acting member;
a gas guide channel having an inlet port for introducing a gas, an outlet port for discharging the gas and a flow path located between the inlet port and the outlet port such that the gas is introduced from the inlet port and flows to the outlet port through an inner space of the flow path; and
a creeping discharge section having a dielectric layer, a discharge electrode and a ground electrode opposed to the discharge electrode with the dielectric layer interposed between the discharge electrode and the ground electrode, the creeping discharge section being disposed such that one of the discharge electrode and the ground electrode faces the flow path directly or via another member and being configured to generate creeping discharge in the flow path with the application of a periodically changing voltage to the discharge electrode,
the gas guide channel being disposed to emit the gas from the outlet port toward the acting member,
the drive section being a high-frequency current supply section that supplies a high-frequency current,
the acting member being configured to allow the acting portion to perform incision action, ablation action or hemostasis action on the biological tissue by flow of the high-frequency current supplied from the high-frequency current supply section through the acting member,
wherein at least a part of the ground electrode is arranged closer to the outlet port than the discharge electrode, and
wherein the acting member, or an assembly member integrally assembled with the acting member, serves as the ground electrode.

The above-mentioned distal device not only enables incision, ablation or hemostasis of the biological tissue by means of the acting portion, but also enables plasma generation at a location closer to the biological tissue. Especially when such an effect is achieved in the case of creeping discharge type, it is possible to irradiate the biological tissue on which the acting portion acts with a greater amount of plasma or irradiate the biological tissue on which the acting portion acts with plasma more efficiently by the application of a lower voltage. It is further possible to, when the acting portion acts on the biological tissue, prevent the biological tissue from being electrically adversely affected. Moreover, the ground electrode disposed in a different region from the acting member or assembly member can be reduced or omitted so that it is possible to simplify the configuration of the distal device in the region different from the acting member or assembly member.

### Effects of the Invention

The present invention provides the effects of irradiating the plasma irradiation target with a greater amount of plasma, or irradiating the plasma irradiation target with plasma more efficiently by the application of a lower voltage, while preventing the plasma irradiation target from being electrically adversely affected.

### Brief Description of Drawings

FIG. 1 is a schematic view of a surgical system equipped with a plasma irradiation apparatus and a distal device according to a first embodiment of the present invention.
FIG. 2 is a schematic enlarged view of a part of the surgical system of FIG. 1 in the vicinity of an acting portion.
FIG. 3 is a schematic perspective view of a structural body of the plasma irradiation apparatus according to the first embodiment.
FIG. 4 is an exploded perspective view of the structural body of FIG. 3 in a state of being divided into three parts.
FIG. 5 is a schematic cross-sectional view of the plasma irradiation apparatus according to the first embodiment, taken, at a center position thereof in a third direction (width direction), along a direction perpendicular to the third direction.
FIG. 6 is a schematic cross-sectional view of the plasma irradiation apparatus according to the first embodiment, taken, at a center position thereof in a first direction, along a direction perpendicular to the first direction.
FIG. 7 is a schematic cross-sectional view of the plasma irradiation apparatus according to the first embodiment, taken, at a center position thereof in a second direction (thickness direction), along a direction perpendicular to the second direction.
FIG. 8 is a schematic view of some process steps for manufacturing of the plasma irradiation apparatus according to the first embodiment.
FIG. 9 is a schematic view of other process steps, carried out subsequent to the process steps of FIG. 8, for manufacturing of the plasma irradiation apparatus according to the first embodiment.
FIG. 10 is a schematic view of a surgical system equipped with a plasma irradiation apparatus and a distal device according to a second embodiment of the present invention.
FIG. 11 is a schematic cross-sectional view of a plasma irradiation apparatus according to a third embodiment of the present invention, taken, at a center position thereof in a second direction (thickness direction), along a direction perpendicular to the second direction.
FIG. 12 is a schematic cross-sectional view of a plasma irradiation apparatus according to another first embodiment example of the present invention, taken, at a center position thereof in a second direction (thickness direction), along a direction perpendicular to the second direction.
FIG. 13 is a schematic cross-sectional view of a plasma irradiation apparatus according to another second embodiment example of the present invention, taken, at a center position thereof in a third direction (width direction), along a direction perpendicular to the third direction.
FIG. 14 is a schematic cross-sectional view of a plasma irradiation apparatus according to another third embodiment example of the present invention, taken, at a center position thereof in a third direction (width direction), along a direction perpendicular to the third direction.
FIG. 15 is a schematic cross-sectional view of a plasma irradiation apparatus according to another fourth embodiment example of the present invention, taken, at a center position thereof in a third direction (width direction), along a direction perpendicular to the third direction.
FIG. 16 is a schematic cross-sectional view of a plasma irradiation apparatus according to another fifth embodiment example of the present invention, taken, at a center position thereof in a third direction (width direction), along a direction perpendicular to the third direction.
FIG. 17 is a schematic view of a plasma irradiation system equipped with a distal device according to another sixth embodiment example of the present invention.

### Description of Embodiments

### <First Embodiment>

### 1. Overall Structure of Surgical System

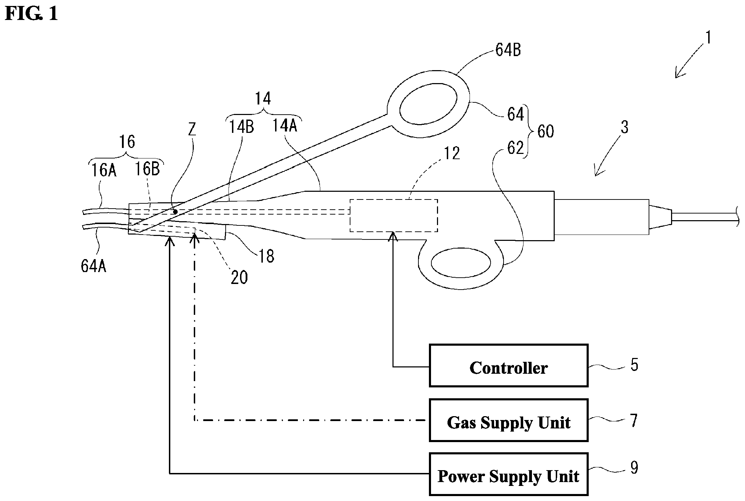

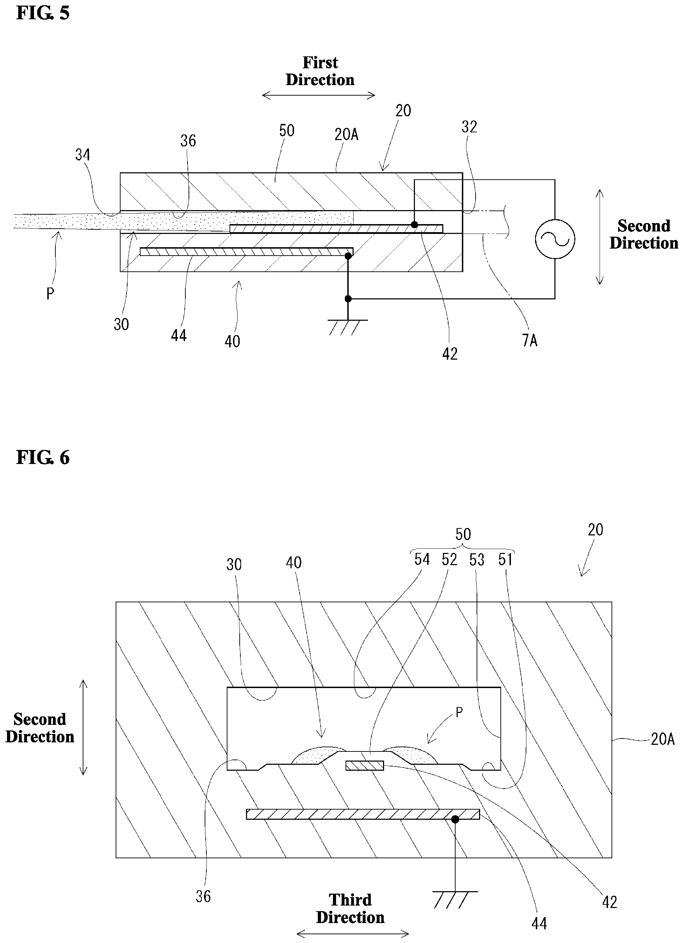

A surgical system 1 according to a first embodiment as shown in FIG. 1 is adapted as a treatment system for performing incision treatment, ablation treatment or hemostasis treatment on biological tissue set as a treatment target. The surgical system 1 mainly includes a distal device 3, a controller 5 that controls an ultrasonic vibration section 12 (as a drive section) of the distal device, a gas supply unit 7 that provides a gas supply to a gas guide channel 30 (see FIG. 6 etc.) inside the distal device 3, a plasma irradiation apparatus 20 and a power supply unit 9 that supplies electric power to the plasma irradiation apparatus 20.

The controller 5 is a device for providing an electric signal to control the ultrasonic vibration section 12 to generate ultrasonic vibration. A flexible signal cable (not shown) is disposed between the distal device 3 and the controller 5 so that the electric signal is sent from the controller 5 to the ultrasonic vibration section 12 through the signal cable.

The gas supply unit 7 is a device for supplying an inert gas such as helium gas, argon gas or the like (hereinafter also simply referred to as "gas"). A flexible gas pipeline (not shown in FIG. 1) is disposed between the distal device 3 and the gas supply unit 7 so that the inert gas is supplied the gas supply unit 7 to the gas guide channel 30 through the gas pipeline. For example, the gas supply unit 7 has a regulator that decompresses highpressure gas supplied from a cylinder etc. and a control section that controls the flow rate of the gas.

The power supply unit 9 is a device for applying a desired voltage between a discharge electrode 42 and a ground electrode 44 of the plasma irradiation apparatus 20. More specifically, the power supply unit 9 applies an alternating voltage with a predetermined frequency between the discharge electrode 42 and the ground electrode 44 while maintaining the ground electrode 44 at a ground potential. The power supply unit 9 can adopt any of various known circuits capable of generating a high voltage (e.g. a high voltage having an amplitude of 0.5 kV to 10 kV) with a high frequency (of e.g. about 20 kHz to 300 kHz). The frequency of the high voltage generated by the power supply unit 9 may be fixed at a constant value or may be varied. Further, the voltage applied between the discharge electrode 42 and the ground electrode 44 by the power supply unit 9 is preferably a periodically changing voltage such as an alternating voltage of sinusoidal waveform or an alternating voltage of nonsinusoidal waveform.

Although the surgical system is exemplified in which the power supply unit 9 for generating an alternating voltage is disposed outside of the distal device 3 in the embodiment of FIG. 1, a power supply circuit for generating an alternating voltage may be disposed inside of the distal device 3 (e.g. in the after-mentioned casing 14 or in the plasma irradiation apparatus 20).

The distal device 3 is a device held and used by an operator who carries out surgical operation. The distal device 3 mainly includes a casing 14, an acting member 16, the plasma irradiation apparatus 20, the ultrasonic vibration section 12 and the like. The casing 14, the acting member 16, the plasma irradiation apparatus 20 and the ultrasonic vibration section 12 are integrated into one as a grip unit (hand grip unit) gripped by the operator. The inert gas and electric power are supplied to this unit through the flexible members.

The casing 14 has a cylindrical shape extending in a predetermined direction, and generally has a base portion 14A and a cylindrical extending portion 14B formed integral with the base portion 14A and extending in the predetermined direction. The ultrasonic vibration section 12 is accommodated in the base portion 14A, whereas the plasma irradiation apparatus 20 is fixed to or integrated with the extending portion 14B.

The ultrasonic vibration section 12 is configured as a known ultrasonic vibrator. When a predetermined electric signal is provided to the ultrasonic vibration section 12 by the controller 5, the ultrasonic vibration section 12 is driven to generate and transmit ultrasonic vibration to the shaft-shaped acting member 16. The ultrasonic vibration section 12 corresponds to one example of the drive section, and drives the acting member 16 in such a manner that incision action, ablation action or thermocoagulation hemostasis action takes place on the biological tissue in the vicinity of an acting portion 16A.

The acting member 16 is a member whose front end part acts as a stationary blade on the biological tissue. The acting member 16 corresponds to an example of a vibratory member to which ultrasonic vibration generated by the ultrasonic vibration section 12 is transmitted. The acting portion 16A is formed on the front end part of the acting member 16. The acting member 16 also has a shaft portion 16B that transmits the vibration from the ultrasonic vibration section 12 to the acting portion 16A. The acting member 16 is hence adapted to cause vibration of the acting portion 16A by transmission of the ultrasonic vibration from the ultrasonic vibration unit 12 to the acting portion 16A through the shaft portion 16B. Accordingly, the acting member 16 is operated to perform incision action, ablation action or hemostasis action on the biological tissue by causing vibration of the acting portion 16A in a state that the acting portion 16A is in close proximity to or in contact with the biological tissue.

The distal device 3 also includes a grip part 60 gripped and used by the operator. The grip part 60 is structured as a movable member displacement mechanism. Any known moving mechanism is employed as the movable member displacement mechanism. The grip part 60 has: a stationary grip portion 62 integrated with the casing 14 by being fixed to the base portion 14A of the casing 14; and a movable member 64 formed in a shaft shape and attached movably relative to the stationary grip portion 62. A movable blade 64Ais provided on one end part of the movable member 64, whereas a movable grip portion 64B is provided on the other end part of the movable member 64. In the grip part 60, the shaft-shaped movable member 64 is pivotable on a pivot axis Z which is located near a front end of the extending portion 14B. When the grip part 60 is operated to bring the movable grip portion 64B closer to the stationary grip portion 62, the movable member 64 pivots in such a manner that the movable blade 64A moves toward the acting portion 16A (stationary blade) of the acting member 16. When the grip part 60 is operated to bring the movable grip portion 64B away from the stationary grip portion 62, the movable member 64 pivots in such a manner that the movable blade 64A moves away from the acting portion 16A (stationary blade).

The above-configured distal device 3 is used to perform incision treatment, ablation treatment or hemostasis treatment on the biological tissue by application of ultrasonic vibration. For example, incision treatment can be performed on the biological tissue by transmitting the ultrasonic vibration to the acting portion 16A in a state that the biological tissue is nipped between the acting portion 16A (stationary blade) and the movable blade 64A. Hemostasis treatment can be performed on the biological tissue through the application of frictional heat by transmitting the ultrasonic vibration to the acting portion 16A in a state that the acting portion 16A is brought into contact with the biological tissue. Further, ablation treatment can be performed on the biological tissue by nipping the biological tissue between the acting portion 16A and the movable blade 64A while transmitting or not transmitting the ultrasonic vibration to the acting member 16. The distal device 3 is also used to, in combination with performing incision treatment, ablation treatment or thermocoagulation hemostasis treatment by application of ultrasonic vibration as described above, perform minimally invasive hemostasis treatment on the biological tissue by irradiation with low-temperature plasma from the plasma irradiation apparatus 20 as will be described later.

### 2. Configuration of Plasma Irradiation Apparatus

Next, the configuration of the plasma irradiation apparatus 20 will be described in detail below.

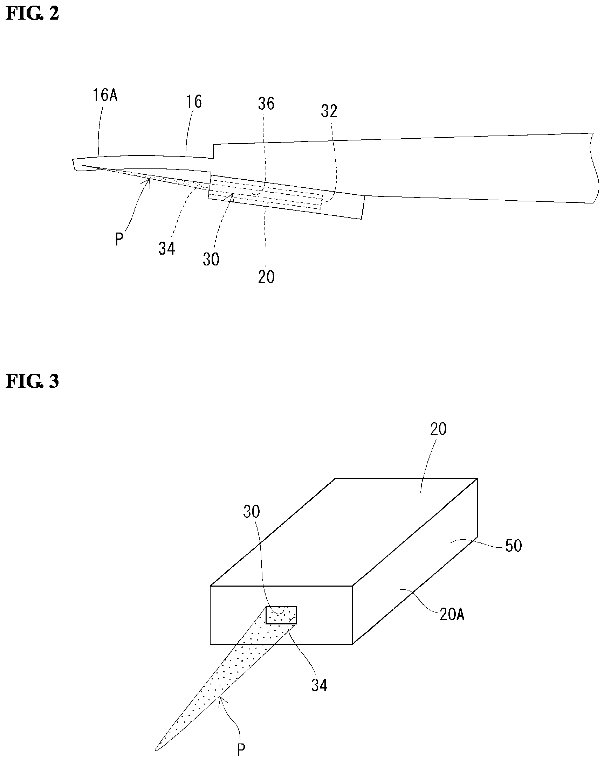

As shown in FIG. 1, the plasma irradiation apparatus 20 is installed as a part of the distal device 3 and is configured to generate dielectric barrier discharge within the distal device 3. In the embodiment of FIG. 1, the plasma irradiation apparatus 20 is fixed to the casing 14 in a state of being held by a holding part 18. The holding part 18, which holds and accommodates therein the plasma irradiation apparatus 20, may be in the form of a casing member having a casing body made of a metal material and covered with a resin coating or a casing member having a casing body made of a resin material and covered with a metal plating. The use of such a holding part contributes to suppression of unintentional discharge or electrical leakage from the plasma irradiation apparatus 20. As shown in FIG. 2, low-temperature plasma generated inside the plasma irradiation apparatus 20 is emitted to the vicinity of the acting portion 16A which is provided on the front end part of the acting member 16.

As shown in FIG. 3, the plasma irradiation apparatus 20 has a structural body 20A of predetermined three-dimensional shape (such as rectangular parallelepiped plate shape). An outlet port 34 is formed in a longitudinal end of the structural body 20A such that the low-temperature plasma P is emitted from the outlet port 34.

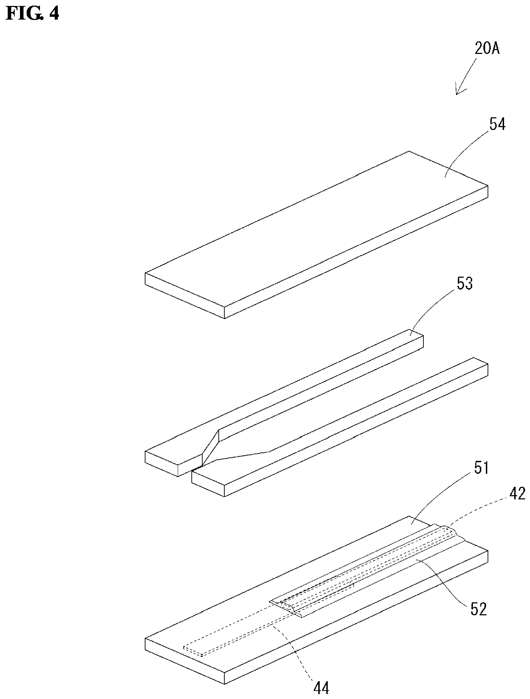

As schematically shown in FIG. 4, the structural body 20A includes: a third dielectric layer 53 located at a center thereof in a thickness direction; a fourth dielectric layer 54 located on one side of the third dielectric layer 53 in the thickness direction; and first and second dielectric layers 51 and 52 located on the other side of the third dielectric layer 53 in the thickness direction. The discharge electrode 42 and the ground electrode 44 are embedded in the dielectric region constituted by the first and second dielectric layers 51 and 52. In FIG. 4, the structural body 20A is schematically shown in exploded perspective view in a state of being divided into three parts. In practice, however, the first dielectric layer 51, the second dielectric layer 52, the third dielectric layer 53 and the fourth dielectric layer 54 are provided as portions of an integral dielectric member 50 (also see FIG. 5).

The plasma irradiation apparatus 20 is generally provided with the gas guide channel 30 and a creeping discharge section 40 as shown in FIG. 5.

The gas guide channel 30 has an inlet port 32 for gas introduction, outlet port 34 for gas discharge and a flow path 36 that extends between the inlet port 32 and the outlet port 34. In other words, the gas guide channel 30 is a gas channel for introducing the inert gas supplied by the gas supply unit 7, which is disposed outside the distal device 3, from the inlet port 32 and guiding the introduced gas to the outlet port 34 through an inner space of the flow path 36. In FIG. 5, a pipeline 7A for guiding the inert gas from the gas supply unit 7 to the inlet port 32 is schematically shown by a two-dot chain line. As shown in FIG. 2, the outlet port 34 of the gas guide channel 30 is arranged at a position adjacent to the acting portion 16A and is directed toward the acting portion 16A such that the acting portion 16A is located on an extension of the inner space of the flow path 36. The gas guide channel 30 is thus adapted to emit the gas from the outlet port 34 toward the acting portion 16A. Consequently, the gas guide channel 30 functions to emit the low-temperature plasma P together with the gas from the outlet port 34 to the acting portion 16A.

As shown in FIG. 5, a direction along which the gas guide channel 30 of the plasma irradiation apparatus 20 extends is defined as a first direction. Among directions perpendicular to the first direction, a thickness direction of the dielectric member 50 is defined as a second direction; and a direction perpendicular to the first and second directions is defined as a third direction (see FIG. 6). In FIG. 5, a longitudinal direction of the structural body 20A in which the dielectric member 50, the discharge electrode 42 and the ground electrode 44 are integrated together corresponds to the first direction. When the structural body 20A is viewed in cross section along a plane perpendicular to the first direction, a short dimension direction of the cross section of the structural body 20A corresponds to the second direction; and a long dimension direction of the cross section of the structural body 20A corresponds to the third direction. In this embodiment, the second direction is a width direction of the structural body 20A; and the third direction is a height direction or thickness direction of the structural body 20A. In the following description, the outlet port 34 side in the first direction is referred to as a front side of the structural body 20A; and the inlet port 32 side in the first direction is referred to as a rear side of the structural body 20A.

The creeping discharge section 40 is constituted by the first dielectric layer 51, the discharge electrode 42 and the ground electrode 44. The discharge electrode 42 and the ground electrode 44 are opposed to each other with the first dielectric layer 51 interposed therebetween. The creeping discharge section 40 functions to develop, in the gas guide channel 30, an electric field based on a potential difference between the discharge electrode 42 and the ground electrode 44 and thereby generate low-temperature plasma discharge as a result of creeping discharge.

More specifically, the creeping discharge section 40 is disposed such that one of the discharge electrode 42 and the ground electrode 44 faces the flow path 36 directly or via another member and is configured to generate creeping discharge by the application of a periodically changing voltage to the discharge electrode 42. The configuration in which "one of the discharge electrode 42 and the ground electrode 44 faces the flow path 36 directly" corresponds to the case where one of the discharge electrode 42 and the ground electrode 44 is exposed inside the inner space of the flow path 36 and constitutes a part of the inner wall of the flow path. The configuration in which "one of the discharge electrode 42 and the ground electrode 44 faces the flow path 36 via another member" corresponds to the case where the one of the discharge electrode 42 and the ground electrode 44 is located at a position near the flow path 36 and is partially or wholly covered by another member. In the latter configuration, the another member constitutes a part of the inner wall of the flow path; and a main surface of the one of the electrodes faces the flow path 36. Although the discharge electrode 42 corresponds to the one of the electrodes and faces the flow path 36 via the another member in the embodiment of FIGS. 5 and 6, the second dielectric layer 52 corresponding to one example of the another member is omitted from illustration in FIG. 5.

As shown in FIG. 6, the creeping discharge section 40 is disposed such that the discharge electrode 42 faces the flow path 36 via a part of the dielectric member 50 (that is, via the second dielectric layer 52). The ground electrode 44 is situated on a side opposite from the flow path 36 with respect to the discharge electrode 42 and is thus located further apart from the flow path 36 than the discharge electrode 42. The creeping discharge section 40 generates low-temperature plasma as a result of creep discharge by the application of a periodically changing voltage to the discharge electrode 42 while maintaining the potential of the ground electrode 44 at a constant reference potential (e.g. a ground potential of 0 V).

As shown in FIG. 6, the dielectric member 50 is provided with the first, second, third and fourth dielectric layers 51, 52, 53 and 54 and formed in a hollow shape as a whole. The first dielectric layer 51 is arranged on one side of the flow path 36 in the second direction (thickness direction), with the ground electrode 44 embedded in the first dielectric layer 51. In other words, the discharge electrode 42 and the ground electrode 44 are opposed to each other via the first dielectric layer 41. The second dielectric layer 52 is made of a ceramic material as a ceramic protective layer and arranged at a position closer to the space of the flow path than the first dielectric layer 51 so as to cover the discharge electrode 42. These first and second dielectric layers 51 and 52 constitute an inner wall of the flow path 36 on one side in the second direction. The fourth dielectric layer 54 is arranged on the other side of the flow path 36 in the second direction (thickness direction), and constitutes an inner wall of the flow path 36 on the other side in the second direction. The third dielectric layer 53 is arranged between the first dielectric layer 51 and the fourth dielectric layer 54 in the second direction, and constitutes side walls of the flow path 36 on one and the other sides in the third direction. Therefore, the flow path 36 is defined by the first, second, third and fourth dielectric layers 51, 52, 53 and 54.

As the materials of the first, second, third and fourth dielectric layers 51, 52, 53 and 54, there can suitably be used a ceramic material such as alumina, a glass material or a resin material. Herein, the use of alumina with high mechanical strength as the material of the dielectric layer facilitates downsizing of the creeping discharge section 40.

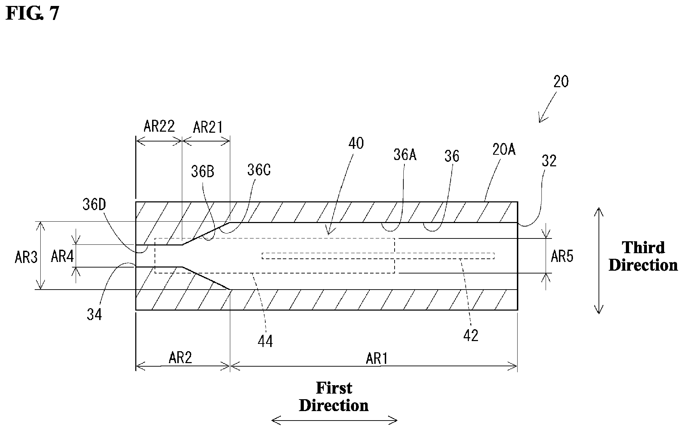

As shown in FIG. 7, the flow path 36 includes: a first flow passage 36A whose space is surrounded at both sides in the second direction and at both sides in the third direction and continues and extends in the first direction; and a second flow passage 36B disposed on a downstream side of the first flow passage 36A. The first flow passage 36A is arranged in a first area AR1 of the structural body 20A in the first direction, whereas the second flow passage 36B is arranged in a second area AR2 of the structural body 20A in the first direction. In FIG. 7, the range of arrangement of the first flow passage 36A in the first direction is indicated as the first area AR1; and the range of arrangement of the second flow passage 36B in the first direction is indicated as the second area AR2.

The first flow passage 36A has an inner circumferential wall surface rectangular in shape (see FIG. 6) when viewed in cross section in a direction perpendicular to the first direction. As shown in FIG. 7, a width of the inner circumferential wall surface of the first flow passage 36A is made constant throughout the entire first area AR1 in the first direction; and a height of the inner circumferential wall surface of the first flow passage 36A is made constant throughout the entire first area AR1 in the first direction.

As shown in FIG. 7, the second flow passage 36B is located closer to the outlet port 34 (downstream side) than the first flow passage 36Ain the first direction and is made narrower in width than the first flow passage 36A. The second flow passage 36 has a reduced width passage portion 36C and a constant width passage portion 36D. The reduced width passage portion 36C is situated within a partial area AR21 in the second area AR2 in the first direction. A width of the inner circumferential wall surface of the reduced width passage portion 36C gradually decreases toward the outlet port 34. A height of the inner circumferential wall surface of the reduced width passage portion 36C is made constant throughout the entire partial area AR21. The constant width passage portion 36D is situated within a partial area AR22 in the second area AR2 in the first direction. A width and height of the inner circumferential wall surface of the constant width passage portion 36D are made constant throughout the entire partial area AR22.

The ground electrode 44 extends linearly in the first direction so as to lie along the flow path 36. For example, the ground electrode 44 is made constant in width and thickness and is disposed within a predetermined area in the first direction. The ground electrode 44 is positioned such that a front end portion of the ground electrode 44 is arranged closer to the outlet port 34 than the discharge electrode 43. More specifically, a part of the ground electrode 44 is situated in the arrangement area AR2 of the second flow passage 36B in the first direction. In the embodiment of FIG. 7, a front end of the ground electrode 44 is located frontward of a front end of the reduced width passage portion 36C (i.e. a rear end of the constant width passage portion 36D). In other words, a part of the ground electrode 44 is situated in the arrangement area AR22 of the constant width passage portion 36D in the first direction. Further, a rear end of the ground electrode 44 is located rearward of a front end of the first flow passage 36A and is located rearward of a front end of the discharge electrode 42 and frontward of a rear end of the discharge electrode 42.

At least a part of the ground electrode 44 (in FIG. 7, the whole of the ground electrode 44) is situated in the arrangement area AR3 of the first flow passage 36A in the third direction. More specifically, at least a part of the ground electrode 44 (in FIG. 7, a part of the ground electrode 44) is situated in the arrangement area AR4 of the constant width passage portion 36D in the third direction and is situated in the formation area of the outlet port 34 in the third direction.

The discharge electrode 42 extends linearly in the first direction so as to lie along the flow path 36. For example, the discharge electrode 42 is made constant in width and thickness and is disposed within a predetermined area in the first direction. More specifically, the discharge electrode 42 is situated only in the arrangement area of the first flow passage 36A in the first direction. That is to say, the discharge electrode 42 is situated only in the first area AR1 among the first and second areas AR1 and AR2 A front end of the discharge electrode 42 is located rearward of a front end of the first flow passage 36A. A rear end of the discharge electrode 42 is located frontward of a rear end of the first flow passage 36A. Further, the width (length in the third direction) of the discharge electrode 42 is made narrower than the width (length in the third direction) of the ground electrode 44. In the embodiment of FIG. 7, the discharge electrode 42 is situated in the arrangement area AR3 of the first flow passage 36A in the third direction. More specifically, the discharge electrode 42 is situated in the arrangement area AR4 of the constant width passage portion 36D in the third direction and is situated in the formation area of the outlet port 34 in the third direction. To be more specific, the discharge electrode 42 is situated in the arrangement area AR5 of the ground electrode 44 in the third direction. An edge of the discharge electrode 42 on one side in the third direction is located closer, than an edge of the ground electrode 44 on one side in the third direction, to the other side in the third direction. An edge of the discharge electrode 42 on the other side in the third direction is located closer to one side in the third direction than an edge of the ground electrode 44 on the other side in the third direction.

The above-configured plasma irradiation apparatus 20 is supplied with an inert gas such that the inert gas flows through the inner space of the flow path 36. Then, an alternating voltage with a predetermined frequency is applied between the discharge electrode 42 and the ground electrode 44 by the power supply unit 9 so as to, for example, while maintaining the ground electrode 44 at a ground potential, oscillate the potential of the discharge electrode 42 within the range between potential values of +A (V) and -A (V) which are respectively higher and lower by a certain degree than the potential of the ground electrode 44. Herein, the value "A" is a positive value. With the application of such an alternating voltage, there occurs a change of electric field between the electrodes in a state that a barrier is formed by the dielectric member 50. As a result, dielectric barrier discharge (more specifically, creeping discharge) is generated in the inner space of the gas guide channel 30. As the inert gas flows to the outlet port 34 in the gas guide channel 30, low-temperature plasma resulting from the creeping discharge is emitted with the gas from the outlet port 34 to the acting portion 16A. Accordingly, when the operator directs the acting portion 16A of the distal device 3 toward e.g. a bleeding site and operates the plasma irradiation apparatus 20, low-temperature plasma is emitted to the bleeding site whereby coagulation of blood can be caused for hemostasis of the bleeding site.

### 3. Manufacturing Method of Plasma Irradiation Apparatus

Next, a manufacturing method of the plasma irradiation apparatus 20 will be described below.

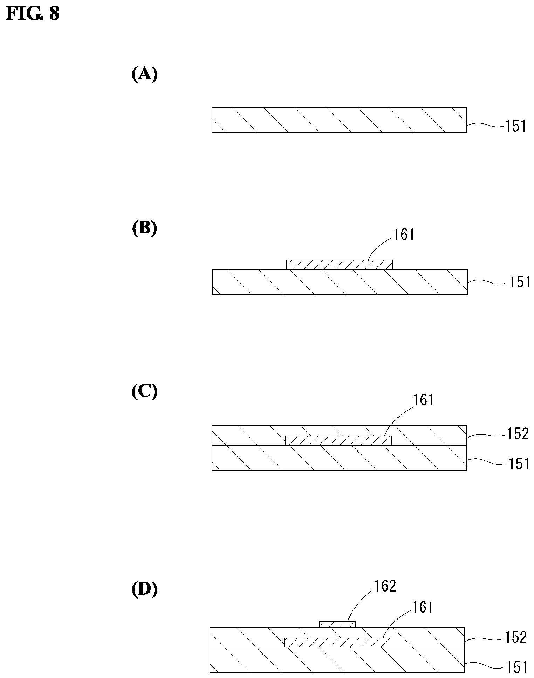

For manufacturing of the plasma irradiation apparatus 20, a first ceramic green sheet forming step and a via-hole forming step are first conducted as shown in FIG. 8(A).

Firstly, the first ceramic green sheet forming step is conducted as follows. In the first ceramic green sheet forming step, a ceramic green sheet of predetermined thickness is formed using a ceramic material predominantly composed of an alumina powder. The ceramic green sheet can be formed by a known technique such as tape molding, extrusion molding etc. After the formation of the ceramic green sheet, the resulting ceramic green sheet is processed by a known processing technique such as laser processing, punching, drilling etc. so as to adjust the outer shape of the ceramic green sheet and form a through hole for a via hole in the ceramic green sheet. After the formation of the through hole for the via hole, a conductive paste (e.g. tungsten paste) is charged into the through hole by a known paste printing machine (not shown) so as to form a green via hole conductor which is to be formed into a conductor part of the via hole. In FIG. 8(A), the first ceramic green sheet 151 after the outer shape processing and the via hole conductor formation is shown; and the green via hole conductor is omitted from illustration.

As shown in FIG. 8(B), a first green conductive layer forming step is conducted after the first ceramic green sheet forming step. In the first green conductive layer forming step, a tungsten paste 161 containing tungsten as a predominant component is applied (printed) by a paste printing machine onto one side of the first ceramic green sheet 151. The tungsten paste 161 corresponds to an example of a first green conductive layer which is to be finally formed into the ground electrode 44.

After the first green conductive layer forming step, a second ceramic green sheet forming step is conducted as shown in FIG. 8(C). In the second ceramic green sheet forming step, a ceramic green sheet of predetermined thickness is formed using a ceramic material predominantly composed of an alumina powder. The resulting ceramic green sheet is subjected to outer shape processing in the same manner as the first ceramic green sheet 151. The thus-obtained second ceramic green sheet 152 after the outer shape processing is placed on and pressure-bonded to the laminate (FIG. 8(B)) obtained in the first green conductive layer forming step.

As shown in FIG. 8(D), a second green conductive layer forming step is conducted after the second ceramic green sheet forming step. In the second green conductive layer forming step, a tungsten paste 162 containing tungsten as a predominant component is applied (printed) by a paste printing machine onto one side of the second ceramic green sheet 152 (FIG. 8(C)) obtained in the second ceramic green sheet forming step.

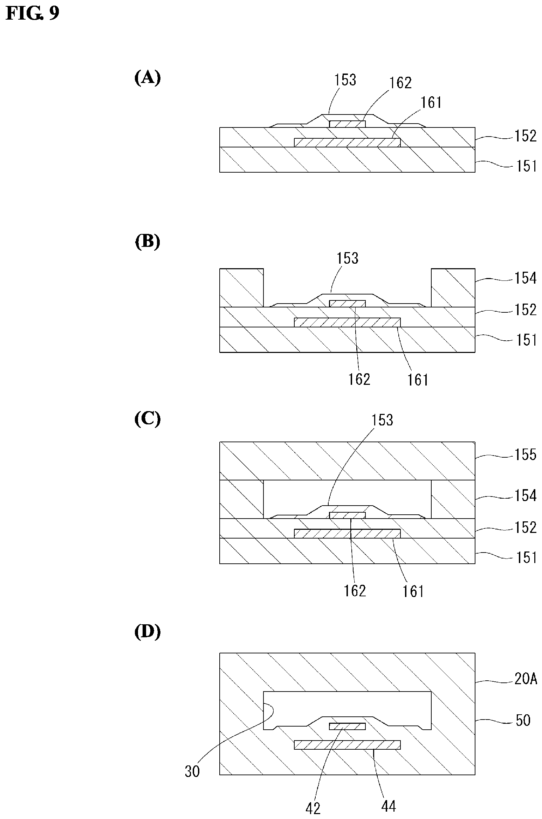

After the second green conductive layer forming step, a ceramic protective layer forming step is conducted as shown in FIG. 9(A). In the ceramic protective layer forming step, an alumina paste 153 is applied (printed) by a paste printing machine onto one side of the laminate (FIG. 8D) obtained in the second green conductive layer forming step in such a manner that the alumina paste 153 covers the tungsten paste 162.

As shown in FIG. 9(B), a third ceramic green sheet forming step is conducted after the third green conductive layer forming step. In the third ceramic green sheet forming step, a ceramic green sheet of predetermined thickness is formed using a ceramic material predominantly composed of an alumina powder. The resulting ceramic green sheet is subjected to outer shape processing in the same manner as the first ceramic green sheet 151. Further, the inner side of the ceramic green sheet is processed to form the gas guide channel in a desired shape. The thus-obtained third ceramic green sheet 154 is placed on and pressure-bonded to the laminate (FIG. 9(A)) obtained in the second green conductive layer forming step.

As shown in FIG. 9(C), a fourth ceramic green sheet forming step is conducted after the third ceramic green sheet forming step. In the fourth ceramic green sheet forming step, a ceramic green sheet of predetermined thickness is formed using a ceramic material predominantly composed of an alumina powder. The resulting ceramic green sheet is subjected to outer shape processing in the same manner as the first ceramic green sheet 151. The thus-obtained fourth ceramic green sheet 155 is placed on and pressure-bonded to the laminate (FIG. 9(B)) obtained in the third ceramic green sheet forming step.

After the fourth ceramic green sheet forming step, the ceramic laminate is subjected to drying and degreasing by known techniques. Subsequently, a firing step is conducted by heating the ceramic laminate (ceramic green sheets and green electrodes) to a predetermined temperature (e.g. about 1400°C to 1600°C) at which alumina and tungsten can be sintered. As a result of the firing step, alumina in the ceramic green sheets, tungsten in the tungsten paste and alumina in the alumina paste are sintered so that there is obtained the structural body 20A (plate-shaped body) where the discharge electrode 42 and the ground electrode 44 are embedded in the dielectric member 50 as shown in FIG. 9(D). Then, a plating part (such as Ni plating part) is applied to the structural body 20A in such a manner that the plating part covers the surface electrode part.

By the method described above, the structural body 20A of planar laminated structure is obtained. This method enables low-cost manufacturing of the plasma irradiation apparatus in a stable shape while suppressing mass production variations of the creeping discharge section 40.

Next, the effects of the above-described configuration will be discussed below.

In the plasma irradiation apparatus 20, at least a part of the ground electrode 44 is arranged closer to the outlet port 34 than the discharge electrode 42. In this arrangement, the ground electrode 44 is brought closer to the outlet port 34; whereas the discharge electrode 42 is brought further away from the outlet port 34. Thus, plasma can be generated in the vicinity of the ground electrode 44 brought closer to the outlet port 34. In other words, plasma is easily generated in an area close to the outlet port 34. Especially when such an effect is achieved in the case of creeping discharge type, the plasma irradiation target (biological tissue) is irradiated with a greater amount of plasma or irradiated with plasma more efficiently by the application of a lower voltage.

Since the discharge electrode 42 is located away from the outlet port 34, the plasma irradiation target (biological tissue) is prevented from being electrically adversely affected by the discharge electrode 42. For example, the electrode (ground electrode 44) brought closer to the outlet port 34 can be maintained at a ground potential of about 0 V and prevented from causing discharge to the plasma irradiation target. Even in the case where the plasma irradiation target (biological tissue) is disposed in the vicinity of the outlet port 34, strong plasma can be prevented from being generated between the discharge electrode 42 and the plasma irradiation target (biological tissue). It is consequently possible to facilitate downsizing of the plasma irradiation apparatus while ensuring low invasiveness.

The flow path 36 of the plasma irradiation apparatus 20 includes: the first flow passage 36A extending in the first direction; and the second flow passage 36B disposed at a location downstream of the first flow passage 36A and closer to the outlet port 34 than the first flow passage 36A in the first direction and having a narrower width than the first flow passage 36A. Thus, the plasma irradiation apparatus 20 allows efficient flow of the gas to the outlet port 34 along the first direction. As the second flow passage 36B of relatively narrow width is arranged downstream of the first flow passage 36A, the flow rate of the gas in the vicinity of the outlet port 34 is increased. It thus becomes easy to, when the outlet port 34 is directed to the plasma irradiation target, efficiently emit the plasma to the plasma irradiation target. This leads to, for example, an increase of the length for which the plasma reaches from the outlet port 34 and easy regulation of the amount of the gas required for discharge.

In the plasma irradiation apparatus 20, at least a part of the ground electrode 44 is situated in the arrangement area AR2 of the second flow passage 36B in the first direction. Since the ground electrode 44 is situated in the arrangement arear AR2 of the relatively narrow width second flow passage 36B, plasma is more easily generated in the second flow passage 36 in which the flow rate of the gas is increased. It thus becomes easy to more efficiently emit the plasma generated in the flow path 36.

Further, the discharge electrode 42 is situated only in the arrangement area AR1 of the first flow passage 36A in the first direction. In other words, the discharge electrode 42 is arranged only in the area relatively far away from the outlet port 34 in the first direction (i.e. in the arrangement area AR1 of the first flow passage 36A) so that, when the outlet port 34 is directed to the plasma irradiation target (biological tissue), the plasma irradiation target is reliably prevented from being electrically adversely affected.

By the way, the plasma irradiation apparatus of creeping discharge type is advantageous in that, at the time of applying a periodically changing voltage to the discharge electrode 42, it is easy to limit the absolute value of the applied voltage. When the absolute value of the applied voltage is too limited, on the other hand, there is a risk that plasma may not be generated favorably. In the plasma irradiation apparatus 20, however, the discharge electrode 42 is made relatively narrow by setting the width of the discharge electrode 42 narrower than the width of the ground electrode 44. With such a configuration, plasma is more easily generated between the discharge electrode 42 and the ground electrode 44 so that it becomes easy to attain both of limitation of the applied voltage and favorable generation of the plasma.

It is further possible, through the use of the common distal device 3, to perform incision treatment, ablation treatment or thermocoagulation hemostasis treatment on the biological tissue by means of the acting portion 16Ato which the ultrasonic vibration is transmitted and to perform minimally invasive hemostasis treatment on the biological tissue by irradiation with the low-temperature plasma. The distal device 3 is hence operable to perform incision and coagulation treatment on the biological tissue (i.e. coagulate the biological tissue while cutting the biological tissue) by means of the acting portion concurrently with performing minimally invasive hemostasis treatment on the biological tissue by irradiation with the low-temperature plasma. In addition, the plasma is easily generated at a location closer to the biological tissue. Especially when such an effect is achieved in the case of creeping discharge type, the biological tissue on which the acting portion 16A acts is irradiated with a greater amount of plasma or irradiated with plasma more efficiently by the application of a lower voltage. Furthermore, the biological tissue is prevented from being electrically adversely affected when the acting portion 16A acts on the biological tissue.

### <Second Embodiment>

A second embodiment will be next described below.

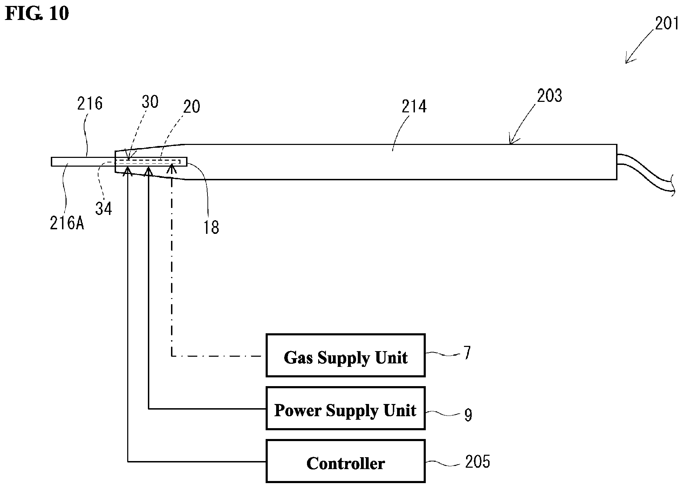

FIG. 10 is a schematic view of a surgical system 201 equipped with a distal device 203 according to the second embodiment. In the following description, the same constituent components of the surgical system 201 with the distal device 203 as those of the surgical system 1 with the distal device 3 of the first embodiment (FIG. 1) are denoted by the same reference numerals; and detailed explanations thereof will be omitted herefrom. For example, the distal device 203 of the surgical system 201 is provided with a plasma irradiation apparatus 20 of the same configuration and function as the plasma irradiation apparatus 20 provided in the distal device 3 of the first embodiment. Further, the surgical system is provided with a gas supply unit 7 and a power supply unit 9 as those provided in the surgical system 1 (FIG. 1).

As shown in FIG. 10, the distal device 203 includes: an acting member 216 having an acting portion that acts on biological tissue; a controller 205 (as a drive section) that drives the acting member 216; and the plasma irradiation apparatus 20. In this embodiment, the plasma irradiation apparatus 20 is also installed as a part of the distal device 203 and fixed to a casing 214 in a state of being held by a holding part 18. The distal device 203 also includes a gas guide channel 30 adapted to allow gas emission from its outlet port 34 toward the acting portion 216A. The casing 214, the acting member 216 and the plasma irradiation apparatus 20 are integrated into one as a grip unit gripped by an operator. An inert gas and electric power are supplied to this unit through flexible members.

The controller 205 is configured as a high-frequency current supply section for supplying a high-frequency current, and corresponds to one example of the drive section. The acting member 216 is formed of e.g. a metal material in a shaft shape and functions as an electrode part through which the high-frequency current supplied from the controller 205 (high-frequency current supply section) flows. The acting member 216 has the function of a conventional electric knife to perform incision action, ablation action or thermocoagulation hemostasis action on the biological tissue by the flow of the high-frequency current through the acting member 216 (electrode part). Although the controller 205 (drive section) is disposed outside of the casing 214 in the embodiment of FIG. 10, the controller 25 can be disposed integral with the casing 241 at a location inside or outside of the casing 214. In other words, the controller 205 can be disposed separately from the plasma irradiation apparatus 20 or disposed integral with the plasma irradiation apparatus 20.

It is possible, through the use of the common distal device 203, to perform incision treatment, ablation treatment or thermocoagulation hemostasis treatment on the biological tissue by flow of high-frequency current through the acting member 216 and to perform minimally invasive hemostasis treatment on the biological tissue by irradiation with low-temperature plasma from the plasma irradiation apparatus 20.

The above-described configuration of the second embodiment provides the same effects as those of the first embodiment.

The distal device 203 not only enables incision, ablation or hemostasis of the biological tissue by means of the acting portion 216, but also enables plasma generation at a location closer to the biological tissue. Especially when such an effect is achieved in the case of creeping discharge type, the biological tissue on which the acting portion 16A acts is irradiated with a greater amount of plasma or irradiated with plasma more efficiently by the application of a lower voltage. Furthermore, the biological tissue is prevented from being electrically adversely affected when the acting portion 216A acts on the biological tissue.

### <Third Embodiment>

A third embodiment will be next described below.

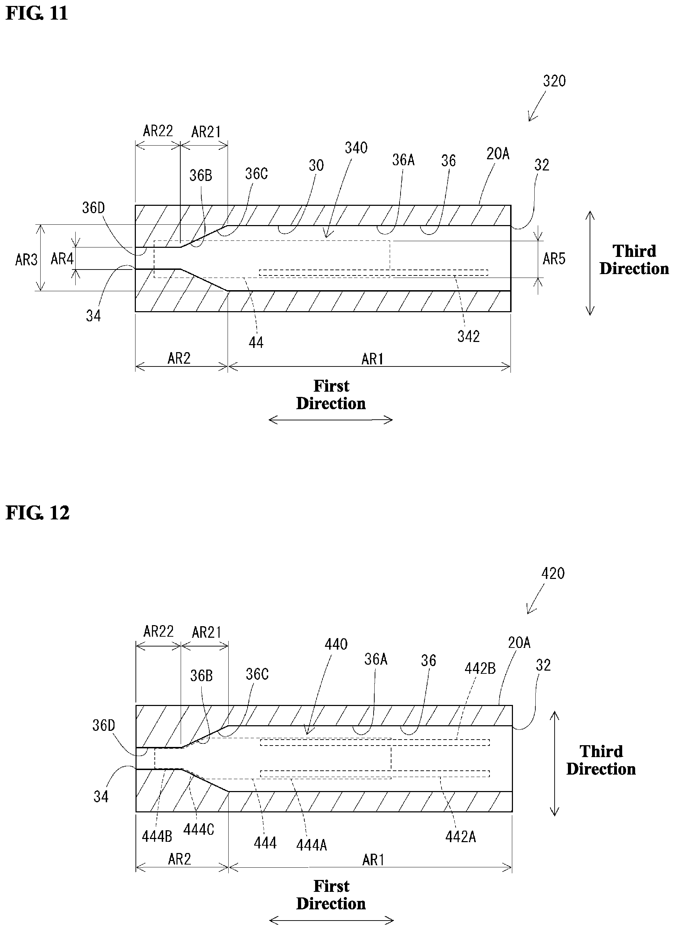

FIG. 11 is a schematic cross-sectional view of a plasma irradiation apparatus 320 according to the third embodiment. Herein, the same constituent components of the plasma irradiation apparatus 320 of FIG. 11 as those of the plasma irradiation apparatus 20 (FIG. 7 etc.) of the first embodiment are denoted by the same reference numerals; and detailed explanations thereof will be omitted herefrom.

The plasma irradiation apparatus 320 of FIG. 11 has a creeping discharge section 340 which is different in configuration from that of the plasma irradiation device 20 of FIG. 7 etc. More specifically, a difference between the plasma irradiation apparatus 320 and the plasma irradiation apparatus 20 of FIG. 7 etc. is that the position of a discharge electrode 342 in the third direction (width direction) is different from the position of the discharge electrode 42 in the third direction. The other structural features of the plasma irradiation apparatus 320 are the same as those of the plasma irradiation apparatus 20 of FIG. 7 etc. The position of the discharge electrode 342 in the second direction (height direction) is the same as that of the discharge electrode 42 (see FIG. 6 etc.). The position of the discharge electrode 342 in the first direction is also the same as that of the discharge electrode 42 (see FIG. 5 etc.). The discharge electrode 342 is covered by a dielectric layer which is similar to the second dielectric layer 52 shown in FIG. 6 etc. so that the dielectric electrode 342 faces a flow path 36 via this dielectric layer.

As shown in FIG. 11, the discharge electrode 342 extends linearly in the first direction so as to lie along the flow path 36. For example, the discharge electrode 342 is made constant in width and thickness and is disposed within a predetermined area in the first direction. More specifically, the discharge electrode 342 is situated only in the arrangement area AR1 of a first flow passage 36A in the first direction. A front end of the discharge electrode 342 is located rearward of a front end of the first flow passage 36A. A rear end of the discharge electrode 342 is located frontward of a rear end of the first flow passage 36A. The width (length in the third direction) of the discharge electrode 342 (i.e. the length of the discharge electrode in the third direction) is made smaller than the width (length in the third direction) of a ground electrode 44. The discharge electrode 342 is also situated in the arrangement area AR3 of the first flow passage 36A in the third direction. When viewed in a plane direction perpendicular to the first direction, however, the arrangement area of the discharge electrode 342 is displaced from the opening area of an outlet port 34. To be more specific, the discharge electrode 342 is out of the formation area AR4 of the outlet port 34 (i.e. the arrangement area of a constant width passage portion 36D). An edge of the discharge electrode 342 on one side in the third direction is located closer to the other side in the third direction than an edge of the ground electrode 44 on one side in the third direction. An edge of the discharge electrode 342 on the other side in the third direction is located closer to one side in the third direction than an edge of the ground electrode on the other side in the third direction.

The above-configured plasma irradiation apparatus 320 provides the same effects as in the first embodiment.

Since the arrangement area of the discharge electrode 342 is displaced from the opening area of the outlet port 34 in the third direction (that is, the discharge electrode 342 is arranged to be hidden when the flow path 36 is seen from the outlet port 34) in the plasma irradiation apparatus 320, the plasma irradiation target is reliably prevented from being electrically adversely affected by the discharge electrode 342 through the inner space of the flow path 36. For example, the electrical adverse influence of the discharge electrode 342 on the plasma irradiation target is prevented reliably even when the plasma irradiation target is disposed in the vicinity of the outlet port 34. It is consequently possible to facilitate downsizing of the plasma irradiation apparatus while ensuring low invasiveness of the plasma irradiation apparatus.

### <Other Embodiments>

The present invention is not limited to the configurations of the embodiments described above with reference to the drawings. For example, the features of some of the embodiments may be combined so long as they are not contradictory to one another. Furthermore, the following examples also fall within the technical scope of the present invention.

A plasma irradiation apparatus 420 as shown in FIG. 12 may be used in place of the plasma irradiation apparatus 320 (FIG. 11) of the third embodiment.

The plasma irradiation apparatus 420 of FIG. 12 is different from the plasma irradiation apparatus 320 of the third embodiment in that a creeping discharge section 440 is used in place of the creeping discharge section 340. More specifically, the plasma irradiation apparatus 420 is different from the plasma irradiation apparatus 320 (FIG. 11) in that: discharge electrodes 442Aand 442B are used in place of the discharge electrode 342; and a ground electrode 444 is used in place of the ground electrode 44. The plasma irradiation apparatus 420 is provided with the discharge electrode 442B as well as the discharge electrode 442A which is similar in configuration to the discharge electrode 342 (FIG. 11). In other words, the plasma irradiation apparatus 420 has a plurality of discharge electrodes 442A and 442B. The arrangement area of each of the discharge electrodes 442A and 442B is displaced from the opening area of an outlet port 34 when viewed in a plane direction perpendicular to the first direction. The ground electrode 444 has a large width portion 444A disposed in the first area AR1 (where a first flow passage 36A is arranged) in the first direction. The large width portion 444A lies astride the first area AR1 and the partial area AR21 in the first direction. A width of the large width portion 444A in the third direction is made constant within a predetermined area in the first direction. In this embodiment example, the width of the large width portion 444A in the third direction is larger than the width of the outlet port 34 in the third direction and is larger than the width of a constant width passage portion 36D in the third direction. Further, an edge of the large width portion 444A on one side in the third direction is located closer to one side in the third direction than an edge of the outlet port 34 on one side in the third direction; and an edge of the large width portion 444A on the other side in the third direction is located closer to the other side in the third direction than an edge of the outlet port 34 on the other side in the third direction. On the other hand, the ground electrode 444 has a small width portion 444B disposed in the partial area AR22 (where the constant width passage portion 36D is arranged) in the first direction. A width of the small width portion 444B in the third direction is smaller than the width of the large width portion 444A in the third direction and is equivalent to the width of the constant width passage portion 36D in the third direction and the width of the outlet port 34 in the third direction. The ground electrode 444 also has a reduced width portion 444C disposed between the large width portion 444A and the small width portion 444B. The reduced width portion 444C is formed such that a width of the reduced width portion 444C in the third direction gradually decreases toward the outlet port 34, and is situated only in a part of the partial area AR21 in the first direction.

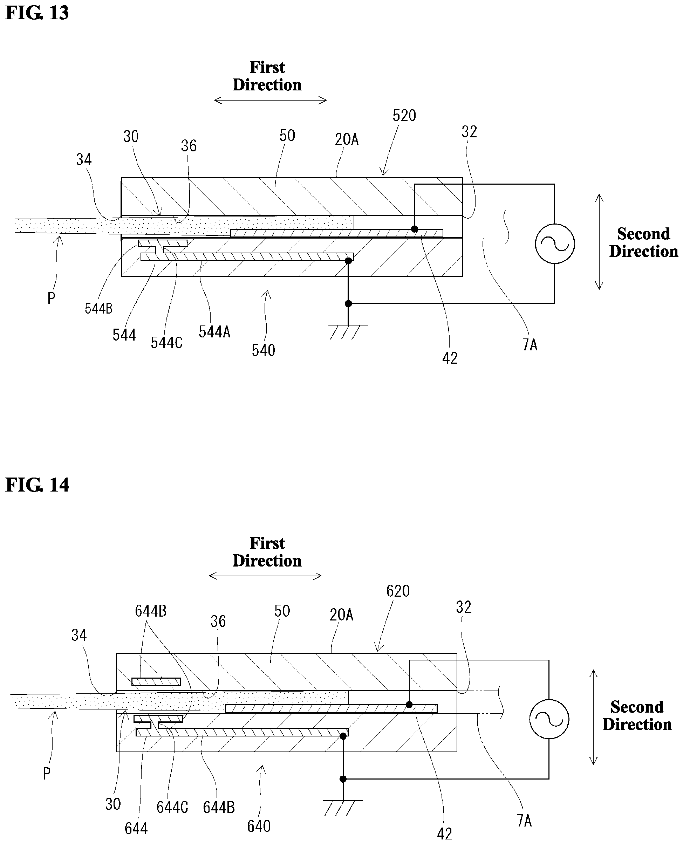

A plasma irradiation apparatus 520 as shown in FIG. 13 may be used in place of the plasma irradiation apparatus 20 (FIG. 5) of the first embodiment.

The plasma irradiation apparatus 520 of FIG. 13 has a creeping discharge section 540, which is different from the creeping discharge section 40 of the plasma irradiation apparatus 20 (FIG. 5) of the first embodiment in that a ground electrode 544 is used in place of the ground electrode 44. As shown in FIG. 13, the ground electrode 544 of the plasma irradiation apparatus 520 includes not only a ground electrode layer 544A which is similar in configuration to the ground electrode 44, but also a ground electrode layer 544B and a conductive portion 544C. The conductive portion 544C functions to make electrical connection between the ground electrode layer 544A and the ground electrode layer 544B. The ground electrode layer 544B is arranged closer to an outlet port 34 than the discharge electrode 42 in the first direction and is arranged closer to the flow path 36 than the ground electrode layer 544A (which is disposed at a position overlapping the discharge electrode 42) in the second direction. With such an arrangement configuration, the ground electrode layer 544B is brought closer the outlet port at a location away from the discharge electrode 42.

Alternatively, a plasma irradiation apparatus 620 as shown in FIG. 14 may be used. The plasma irradiation apparatus 620 of FIG. 14 is similar to that of FIG. 13 except for the configuration of a creeping discharge section 640. More specifically, the plasma irradiation apparatus 620 is different from the plasma irradiation apparatus of FIG. 13 in that a ground electrode portion 644B is used in place of the ground electrode layer 544B. In the ground electrode 644 of the plasma irradiation apparatus 620, the ground electrode portion 644B is provided as an annular electrode layer that surrounds a flow path 36. Herein, a ground electrode layer 644A of the ground electrode 644 is similar to the ground electrode layer 544A of FIG. 13; and a conductive portion 644C of the ground electrode 644 is similar to the conductive portion 544C of FIG. 13.

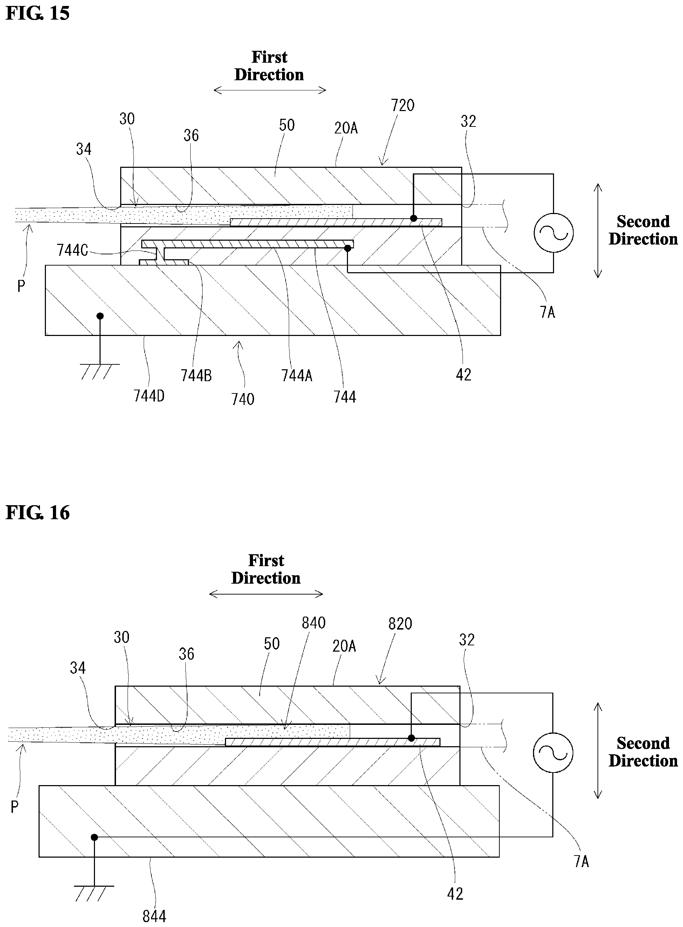

In the distal device 3 (FIG. 1) of the first embodiment, the distal device 201 (FIG. 10) of the second embodiment or the other distal device described above, a plasma irradiation apparatus 720 as shown in FIG. 15 may be used in place of the plasma irradiation apparatus 20. The plasma irradiation apparatus 720 of FIG. 15 is different in configuration from the plasma irradiation apparatus 20 of FIG. 5 etc. in that a creeping discharge section 740 is used in place of the creeping discharge section 40. More specifically, the plasma irradiation apparatus 720 has a ground electrode 744 in place of the ground electrode 44 (FIG. 5). The ground electrode 744 includes not only a ground electrode layer 744A which is similar in configuration to the ground electrode 44, but also a ground electrode portion 744D, a ground electrode layer 744B electrically connected to the ground electrode portion 744D and a conductive portion 744C that electrically connects the ground electrode layer 744A and the ground electrode layer 744B to each other. Each of the ground electrode portion 744D, the ground electrode layers 744A and 744B and the conductive portion 744C is maintained at a ground potential. The ground electrode portion 744D can be the acting member of any of the above-mentioned distal devices or the assembly member (such as casing) assembled with the acting member of any of the above-mentioned distal devices. In the embodiment example of FIG. 15, the ground electrode portion 744D (i.e. the acting member or the assembly member integrally assembled with the acting member) is provided as a part of the ground electrode and maintained at a ground potential during the application of a high-frequency voltage. As shown in FIG. 15, a part of the ground electrode portion 744D is arranged closer to an outlet port 34 than the discharge electrode 42. To be more specific, the ground electrode portion 744D extends to a point frontward of the outlet port 34 in the first direction. With this configuration, it is possible to facilitate wiring simplification of the ground electrode.

In the distal device 3 (FIG. 1) of the first embodiment, the distal device 201 (FIG. 10) of the second embodiment or the other distal device described above, a plasma irradiation apparatus 820 as shown in FIG. 16 may be used in place of the plasma irradiation apparatus 20. The plasma irradiation apparatus 820 of FIG. 16 is different in configuration from the plasma irradiation apparatus 20 of FIG. 5 etc. in that a creeping discharge section 840 is used in place of the creeping discharge section 40. More specifically, the plasma irradiation apparatus 820 of FIG. 16 is different in configuration from the plasma irradiation apparatus 20 of FIG. 5 etc. in that a ground electrode 844 is used with omission of the ground electrode 44 (FIG. 5). The other structural features of the plasma irradiation apparatus 820 are the same as those of the plasma irradiation apparatus 20 of FIG. 5 etc. In the embodiment example of FIG. 16, the ground electrode 844 can be the acting member of any of the above-mentioned distal devices or the assembly member (such as casing) assembled with the acting member of any of the above-mentioned distal devices. In FIG. 16, the ground electrode 844 serves as not only the ground electrode but also "the acting member or the assembly member assembled integrally with the acting member", and is maintained at a ground potential during the application of a high-frequency voltage. As shown in FIG. 16, a part of the ground electrode 844 is arranged closer to an outlet port 34 than the discharge electrode 42. To be more specific, the ground electrode 844 extends to a point frontward of the outlet port 34 in the first direction. In this case, the ground electrode disposed in a different region from the acting member or the assembly member can be reduced or omitted. It is thus possible to simplify the configuration of the distal device in the region different from the acting member or assembly member. It is further possible to facilitate wiring simplification of the ground electrode.

Although a part of the ground electrode is situated in the arrangement area of the second flow passage in the first direction in the above-described embodiments, the whole of the ground electrode may be situated in the arrangement area of the second flow passage in the first direction.

In the above-described first embodiment, the distal device is provided by assembling therein the plasma irradiation apparatus so as to function as an ultrasonic knife. The distal device may alternatively be provided by assembling the plasma irradiation apparatus to any known surgical instrument (such as scalpel, forceps etc.) with no electrical function.

In some of the above-described embodiments, the drive section is disposed inside of the casing which constitutes a part of the distal device (more specifically, the casing in which the acting member is installed). Alternatively, the drive section may be disposed outside of the casing. Even in the case where the drive section is disposed outside of the casing, the drive section can be regarded as a part of the distal device.

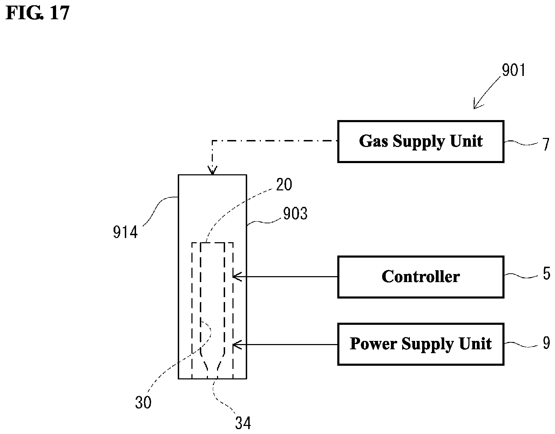

Although the plasma irradiation apparatus is installed as a part of the distal device in the above-described embodiments, the plasma irradiation apparatus may not be installed as a part of the distal device. In other words, the plasma irradiation apparatus can be used solely. For example, it is feasible to assemble the plasma irradiation apparatus 20, the controller 5, the gas supply unit 7 and the power supply unit 9 into a plasma irradiation system 901 as shown in FIG. 17 by omitting some part from the configuration of FIG. 1. The plasma irradiation system 901 is applicable as a surgical system or applicable for any use other than surgical use. In the embodiment example of FIG. 17, a distal device 903 is provided in which the plasma irradiation apparatus 20 is installed in the casing 914 as in the case of the first embodiment. The casing 914 and the plasma irradiation apparatus 20 are integrated as a grip unit (hand grip part). In this case, the controller 5 is preferably configured to perform the other control instead of the control of the ultrasonic vibration section 12. Even in such a configuration, a flexible pipeline is preferably provided between the gas supply unit 7 and the plasma irradiation apparatus 20. The power supply unit 9 may be disposed inside of the casing 914 or outside of the casing 914.

In the claims and specification, the expression "acting on biological tissue" means that the acting member exerts an influence on the biological tissue to perform at least one of incision treatment, ablation treatment and hemostasis treatment. The acting members exemplified in the above-described embodiments are merely examples. The acting member can employ various structures other than those in the above-described embodiments as long as the acting member exerts an influence on the biological tissue to perform at least one of incision treatment, ablation treatment and hemostasis treatment.

### Description of Reference Numerals

1, 201: Surgical system
3, 203, 903: Distal device
12: Ultrasonic vibration section (Drive section)
16, 216: Acting member
16A, 216A: Acting portion
20, 320, 420, 520, 620, 720, 820: Plasma irradiation apparatus
30: Gas guide channel
32: Inlet port
34: Outlet port
36: Flow path
36A: First flow passage
36B: Second flow passage
40, 340, 440, 540, 640, 740, 840: Creeping discharge section
42, 342, 442A, 442B: Discharge electrode
44, 444, 544, 644, 744, 844: Ground electrode
51: First dielectric layer (Dielectric layer)
205: Controller (Drive section)

## Claims

1. A plasma irradiation apparatus, comprising:
a gas guide channel having an inlet port for introducing a gas, an outlet port for discharging the gas and a flow path located between the inlet port and the outlet port such that the gas introduced from the inlet port flows to the outlet port through an inner space of the flow path; and
a creeping discharge section having a dielectric layer, a discharge electrode and a ground electrode opposed to the discharge electrode with the dielectric layer interposed between the discharge electrode and the ground electrode, the creeping discharge section being disposed such that one of the discharge electrode and the ground electrode faces the flow path directly or via another member and being configured to generate creeping discharge in the flow path by the application of a periodically changing voltage to the discharge electrode,
wherein at least a part of the ground electrode is arranged closer to the outlet port than the discharge electrode.

2. The plasma irradiation apparatus according to claim 1,
wherein the gas guide channel has the outlet port on one side thereof in a first direction and the inlet port on the other side thereof in the first direction,
wherein the flow path includes: a first flow passage extending along the first direction; and a second flow passage disposed at a location downstream of the first flow passage and closer to the outlet port than the first flow passage in the first direction and having a width narrower than that of the first flow passage.

3. The plasma irradiation apparatus according to claim 2,
wherein at least a part of the ground electrode is situated in an arrangement area of the second flow passage in the first direction.

4. The plasma irradiation apparatus according to claim 3,
wherein the discharge electrode is situated only in an arrangement area of the first flow passage in the first direction.

5. The plasma irradiation apparatus according to any one of claims 2 to 4,
wherein the discharge electrode faces the flow path directly or via another member, and
wherein, in a plane direction perpendicular to the first direction, an arrangement area of the discharge electrode is displaced from an opening area of the outlet port.

6. The plasma irradiation apparatus according to any one of claims 1 to 5,
wherein a width of the discharge electrode is narrower than a width of the ground electrode.

7. A distal device, comprising:
an acting member having an acting portion that acts on biological tissue; and
the plasma irradiation apparatus according to any one of claims 1 to 6,
wherein the gas guide channel is disposed to emit the gas from the outlet port toward the acting portion.

8. A distal device, comprising:
an acting member having an acting portion that acts on biological tissue;
a drive section that drives the acting member; and
the plasma irradiation apparatus according to any one of claims 1 to 6,
wherein the gas guide channel is disposed to emit the gas from the outlet port toward the acting portion,
wherein the drive section is an ultrasonic vibration section that generates ultrasonic vibration, and
wherein the acting member is configured to, by transmission of the ultrasonic vibration from the ultrasonic vibration section to the acting member, allow the acting portion to vibrate and thereby perform incision action, ablation action or hemostasis action on the biological tissue.

9. A distal device, comprising:
an acting member having an acting portion that acts on biological tissue;
a drive section that drives the acting member; and
the plasma irradiation apparatus according to any one of claims 1 to 6,
wherein the gas guide channel is disposed to emit the gas from the outlet port toward the acting portion,
wherein the drive section is a high-frequency current supply section that supplies a high-frequency current, and
wherein the acting member is configured to, by flow of the high-frequency current from the high-frequency current supply section through the acting member, allow the acting portion to perform incision action, ablation action or hemostasis action on the biological tissue.

10. The distal device according to any one of claims 7 to 9,
wherein the acting member, or an assembly member integrally assembled with the acting member, serves as the ground electrode.

11. A distal device, comprising:
an acting member having an acting portion that acts on biological tissue;
a gas guide channel having an inlet port for introducing a gas, an outlet port for discharging the gas and a flow path located between the inlet port and the outlet port such that the gas is introduced from the inlet port and flows to the outlet port through an inner space of the flow path; and
a creeping discharge section having a dielectric layer, a discharge electrode and a ground electrode opposed to the discharge electrode with the dielectric layer interposed between the discharge electrode and the ground electrode, the creeping discharge section being disposed such that one of the discharge electrode and the ground electrode faces the flow path directly or via another member and being configured to generate creeping discharge in the flow path by the application of a periodically changing voltage to the discharge electrode,
the gas guide channel being disposed to emit the gas from the outlet port toward the acting portion,
wherein at least a part of the ground electrode is arranged closer to the outlet port than the discharge electrode, and
wherein the acting member, or an assembly member integrally assembled with the acting member, serves as the ground electrode.

12. A distal device, comprising:
an acting member having an acting portion that acts on biological tissue;
a drive section that drives the acting member;
a gas guide channel having an inlet port for introducing a gas, an outlet port for discharging the gas and a flow path located between the inlet port and the outlet port such that the gas is introduced from the inlet port and flows to the outlet port through an inner space of the flow path; and
a creeping discharge section having a dielectric layer, a discharge electrode and a ground electrode opposed to the discharge electrode with the dielectric layer interposed between the discharge electrode and the ground electrode, the creeping discharge section being disposed such that one of the discharge electrode and the ground electrode faces the flow path directly or via another member and being configured to generate creeping discharge in the flow path by the application of a periodically changing voltage to the discharge electrode,
the gas guide channel being disposed to emit the gas from the outlet port toward the acting portion,
the drive section being an ultrasonic vibration section that generates ultrasonic vibration,
the acting member being configured to, by transmission of the ultrasonic vibration from the ultrasonic vibration section to the acting member, allow the acting portion to vibrate and thereby perform incision action, ablation action or hemostasis action on the biological tissue,
wherein at least a part of the ground electrode is arranged closer to the outlet port than the discharge electrode, and
wherein the acting member, or an assembly member integrally assembled with the acting member, serves as the ground electrode.

13. A distal device, comprising:
an acting member having an acting portion that acts on biological tissue;
a drive section that drives the acting member;
a gas guide channel having an inlet port for introducing a gas, an outlet port for discharging the gas and a flow path located between the inlet port and the outlet port such that the gas is introduced from the inlet port and flows to the outlet port through an inner space of the flow path; and
a creeping discharge section having a dielectric layer, a discharge electrode and a ground electrode opposed to the discharge electrode with the dielectric layer interposed between the discharge electrode and the ground electrode, the creeping discharge section being disposed such that one of the discharge electrode and the ground electrode faces the flow path directly or via another member and being configured to generate creeping discharge in the flow path by the application of a periodically changing voltage,
the gas guide channel being disposed to emit the gas from the outlet port toward the acting portion,
the drive section being a high-frequency current supply section that supplies a high-frequency current,
the acting member being configured to, by flow of the high-frequency current from the high-frequency current supply section through the acting member, allow the acting portion to perform incision action, ablation action or hemostasis action on the biological tissue,
wherein at least a part of the ground electrode is arranged closer to the outlet port than the discharge electrode, and
wherein the acting member, or an assembly member integrally assembled with the acting member, serves as the ground electrode.
